(19) Europäisches Patentamt / European Patent Office / Office européen des brevets

(11) **EP 4 516 922 A1**

(12) **EUROPEAN PATENT APPLICATION**
published in accordance with Art. 153(4) EPC

(43) Date of publication:
**05.03.2025 Bulletin 2025/10**

(21) Application number: **23796488.7**

(22) Date of filing: **27.04.2023**

(51) International Patent Classification (IPC):
*C12Q 1/06* (2006.01)   *A61B 5/055* (2006.01)
*C12M 1/34* (2006.01)   *G01N 33/48* (2006.01)
*G01N 33/92* (2006.01)   *G16H 30/00* (2018.01)

(52) Cooperative Patent Classification (CPC):
A61B 5/055; C12M 1/34; C12Q 1/06; G01N 33/48;
G01N 33/92; G16H 30/00

(86) International application number:
**PCT/JP2023/016673**

(87) International publication number:
**WO 2023/210752 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.04.2022 JP 2022074111**

(71) Applicant: **OSAKA UNIVERSITY**
**Suita-shi**
**Osaka 565-0871 (JP)**

(72) Inventors:
• **KODAMA Takahiro**
  **Suita-shi, Osaka 565-0871 (JP)**
• **MURAI Hiroki**
  **Suita-shi, Osaka 565-0871 (JP)**
• **TAKEHARA Tetsuo**
  **Suita-shi, Osaka 565-0871 (JP)**

(74) Representative: **Arnold & Siedsma**
**Bezuidenhoutseweg 57**
**2594 AC The Hague (NL)**

(54) **METHOD FOR TESTING SUSCEPTIBILITY TO IMMUNE CHECKPOINT INHIBITOR**

(57) An object is to provide a biomarker serving as an indicator for susceptibility to an immune checkpoint inhibitor for liver cancer, to thereby provide appropriate treatment for an individual patient with liver cancer. Patients with liver cancer have been stratified according to their prognosis or tumor immune microenvironments, and it has been found that a new link lies between steatotic liver cancer and an immune-enriched but immune-exhausted tumor immune microenvironment. It has been further found that patients with steatotic liver cancer each have susceptibility to immunotherapy using an immune checkpoint inhibitor. The use of a fat fraction in a liver cancer tissue as an indicator for the susceptibility to the immune checkpoint inhibitor enables prediction of efficacy of the immune checkpoint inhibitor, and thus enables provision of appropriate treatment for an individual patient with liver cancer. The fat fraction may be calculated from an image of the liver cancer tissue or a signal for displaying the image.

FIG.13

| Antitumor effect | non-Steatotic HCC | Steatotic HCC | *P* value |
|---|---|---|---|
| CR/PR/SD/PD | 0/6/7/10 | 0/2/5/0 | |
| DCR (%) | 56.5 | 100 | 0.064 |

EP 4 516 922 A1

**Description**

The present invention relates to a method of testing susceptibility to an immune checkpoint inhibitor, an apparatus for testing susceptibility to an immune checkpoint inhibitor, and a program for testing susceptibility to an immune checkpoint inhibitor.

The present application claims priority from Japanese Patent Application No. 2022-074111, which is incorporated herein by reference.

Background Art

Liver cancer is the fourth leading cause of cancer-related death worldwide (Non Patent Literature 1). Immunotherapy has become the standard-of-care treatment for hepatocellular carcinoma (HCC), but its efficacy remains limited. Hepatocellular carcinoma is the most common type of primary liver cancer, and is a heterogeneous disease with a variety of etiological factors (Non Patent Literatures 2 and 3). Hepatitis C virus (HCV) is one of the major causes of hepatocellular carcinoma, and the prevalence of HCV-related hepatocellular carcinoma (HCV-HCC) has been decreasing worldwide owing to recent advances in surveillance and treatment (Non Patent Literature 4). Meanwhile, the prevalence of nonviral hepatocellular carcinoma is increasing rapidly, and it has various causes, such as heavy drinking, nonalcoholic fatty liver disease (NAFLD), and diabetes mellitus (DM) (Non Patent Literature 5). Understanding hepatocellular carcinoma diversity to develop targeted therapies requires unravelling the molecular mechanism underlying the carcinogenesis process. To this end, profiling of hepatocellular carcinoma at genetic and transcriptomic levels has been performed (Non Patent Literatures 6 to 8). However, the relationships between molecular features and clinicopathological features in nonviral hepatocellular carcinoma have not been fully characterized.

In recent years, immune checkpoint inhibitors (ICIs) have shown remarkable efficacy in various kinds of solid cancers (Non Patent Literature 9). Those immune checkpoint inhibitors include monoclonal antibodies directed against cytotoxic T-lymphocyte-associated protein 4 (CTLA-4), programmed cell death protein 1 (PD-1), and its ligand PD-L1 (CD274). In 2020, the IMbrave150 trial showed that atezolizumab (anti-PD-L1 antibody) plus bevacizumab (anti-VEGF antibody) therapy significantly prolonged a progression-free survival rate (PFS) and an overall survival rate (OS) compared with sorafenib in patients with unresectable HCC (Non Patent Literature 10), and combined immunotherapy has currently been in the spotlight in the HCC field. Although the atezolizumab plus bevacizumab therapy has become a first-line drug for patients with advanced hepatocellular carcinoma, many patients will not derive much of a benefit under the current circumstances. Meanwhile, when the drug of choice is ineffective, it is required to change a treatment method, for example, by changing the drug. However, when such changing is repeated, functional hepatic reserve is gradually reduced. Accordingly, it is important to choose an appropriate drug for each patient.

In general, the tumor immune microenvironments (TIMEs) are stratified into immune-excluded, immune-active, and immune-exhausted subtypes based on the levels of tumor-infiltrating lymphocytes (TILs) and immune checkpoint expression (Non Patent Literatures 11 and 12). A meta-analysis showed that TIL levels and PD-1/PD-L1 expression are positively associated with the response to ICIs in a variety of cancer types (Non Patent Literatures 13 and 14). However, the heterogeneity of tumor immunity in hepatocellular carcinoma, particularly in nonviral hepatocellular carcinoma, and its effect on the response to combined immunotherapy have not been clarified.

Citation List

Non Patent Literature

[NPL 1] CA Cancer J Clin. 2018 Nov; 68(6): 394-424.
[NPL 2] Recent Results Cancer Res. 2013; 190: 21-32.
[NPL 3] Gastroenterology. 2019 Jul; 157(1): 54-64.
[NPL 4] Liver Int. 2013 Oct; 33(9): 1420-7
[NPL 5] World J Hepatol. 2013 Jun 27; 5(6): 311-322.
[NPL 6] Nat Genet. 2015 May; 47(5): 505-511.
[NPL 7] Cancer Res. 2009 Sep 15; 69(18): 7385-92.
[NPL 8] Cancer Res. 2008 Aug 15; 68(16): 6779-88.
[NPL 9] Immunotherapy. 2016 Jun; 8(7): 821-37.
[NPL 10] N Engl J Med. 2020 May 14; 382(20): 1894-1905.
[NPL 11] J Hepatol. 2020 Feb; 72(2): 215-229.
[NPL 12] Gastroenterology. 2017 Sep; 153(3): 812-826.

[NPL 13] EClinical Medicine. 2021 Sep 16; 41: 101134.
[NPL 14] JAMA Oncol. 2019 Aug 1; 5(8): 1195-1204.

Summary of Invention

Technical Problem

In view of low efficacy of immunotherapy with an immune checkpoint inhibitor in pharmacotherapy of liver cancer, an object of the present invention is to provide a biomarker serving as an indicator for susceptibility to an immune checkpoint inhibitor.

Solution to Problem

In order to achieve the above-mentioned object, the inventors of the present invention have focused attention on high heterogeneity of molecular abnormalities and tumor immune microenvironments in patients with liver cancer. The inventors have repeated extensive investigations, stratified patients with liver cancer according to their prognosis or tumor immune microenvironments, and as a result, identified a new link between steatotic liver cancer and an immune-enriched but immune-exhausted tumor immune microenvironment. The inventors have further found that patients with steatotic liver cancer each have susceptibility to immunotherapy using an immune checkpoint inhibitor, and completed the present invention.

That is, the present invention includes the following.

1. A method of testing susceptibility to an immune checkpoint inhibitor, comprising a step of measuring a fat fraction in a liver cancer tissue.

2. The method according to the above-mentioned item 1, wherein the fat fraction is calculated from an image obtained from the liver cancer tissue or a signal for displaying the image obtained from the liver cancer tissue.

3. The method according to the above-mentioned item 2, wherein the image is an MRI image.

4. The method according to the above-mentioned item 3, wherein the MRI image is a chemical-shift imaging image.

5. The method according to any one of the above-mentioned items 1 to 4, further comprising a step of comparing the fat fraction with a reference value, wherein a case in which the fat fraction is equal to or higher than the reference value is determined as having high susceptibility to the immune checkpoint inhibitor or having susceptibility to the immune checkpoint inhibitor.

6. The method according to the above-mentioned item 5, wherein the reference value is a value selected from a range of 5% or more and 15% or less.

7. The method according to the above-mentioned item 6, wherein the reference value for the fat fraction measured by chemical-shift imaging is 10%.

8. The method according to any one of the above-mentioned items 1 to 7, wherein the immune checkpoint inhibitor is an anti-PD-L1 antibody or an anti-PD-1 antibody.

9. The method according to any one of the above-mentioned items 1 to 8, wherein the liver cancer tissue is a hepatocellular carcinoma tissue.

10. An apparatus for testing susceptibility to an immune checkpoint inhibitor, comprising:

a signal detection unit configured to detect an MR signal of a liver cancer tissue;
a fat fraction calculation unit configured to calculate a fat fraction in the liver cancer tissue from the MR signal having been detected; and
an output unit configured to output a susceptibility level to the immune checkpoint inhibitor based on comparison between the fat fraction having been calculated and a reference value.

11. A program for testing susceptibility to an immune checkpoint inhibitor, the program causing a computer to execute the steps of:

inputting data on an MR signal of a liver cancer tissue;
calculating a fat fraction in the liver cancer tissue from the data on the MR signal having been input; and
outputting a susceptibility level based on comparison between the fat fraction having been calculated and a reference value.

Advantageous Effects of Invention

The present invention enables prediction of the efficacy of an immune checkpoint inhibitor, and thus enables choice of an appropriate treatment method for an individual patient with liver cancer.

Most patients with liver cancer undergo diagnostic imaging without undergoing tumor biopsy in general practice, and hence the method, the apparatus, and the program of the present invention are easily clinically applicable. In addition, the test method of the present invention is a test method with less stress on the patients.

Brief Description of Drawings

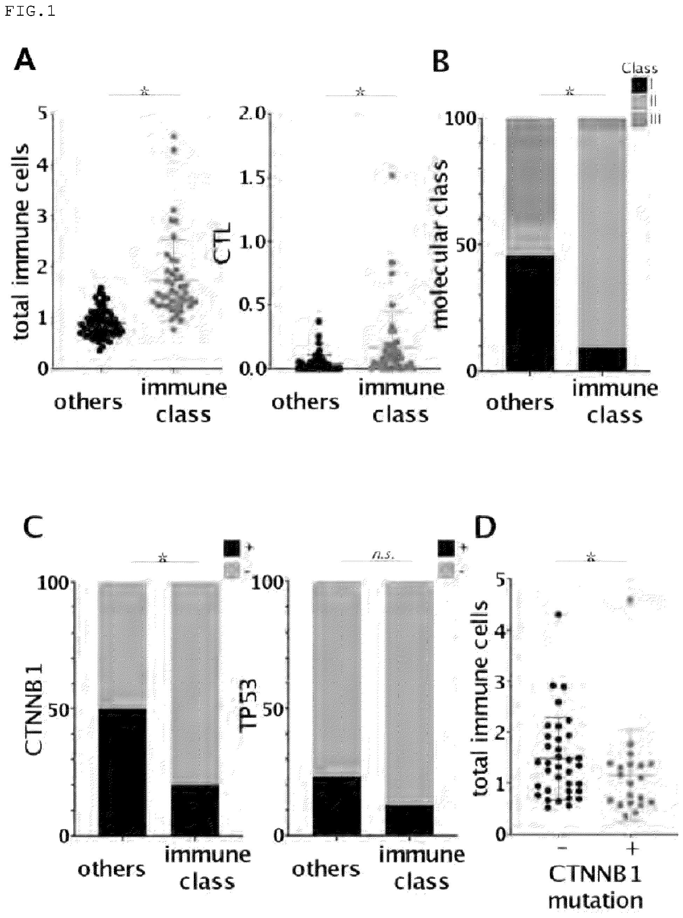

FIGS. 1 show the results of classification of nonviral hepatocellular carcinomas based on tumor immune microenvironments. FIG. 1A shows the results of comparison of CIBERSORT scores for total immune cells and cytotoxic T cells between an immune class and the other class, FIG. 1B shows the results of comparison of the percentages of molecular classes between the immune class and the other class, FIG. 1C shows the results of comparison of the percentages of CTNNB1 or TP53 mutations between the immune class and the other class, and FIG. 1D shows the results of comparison of CIBERSORT scores for total immune cells between hepatocellular carcinoma with CTNNB1 mutation ("+" in the figure) and hepatocellular carcinoma without CTNNB1 mutation ("-" in the figure). In the figures, the "immune class" means the immune class, and the "others" means the other class. (Test Example 2)

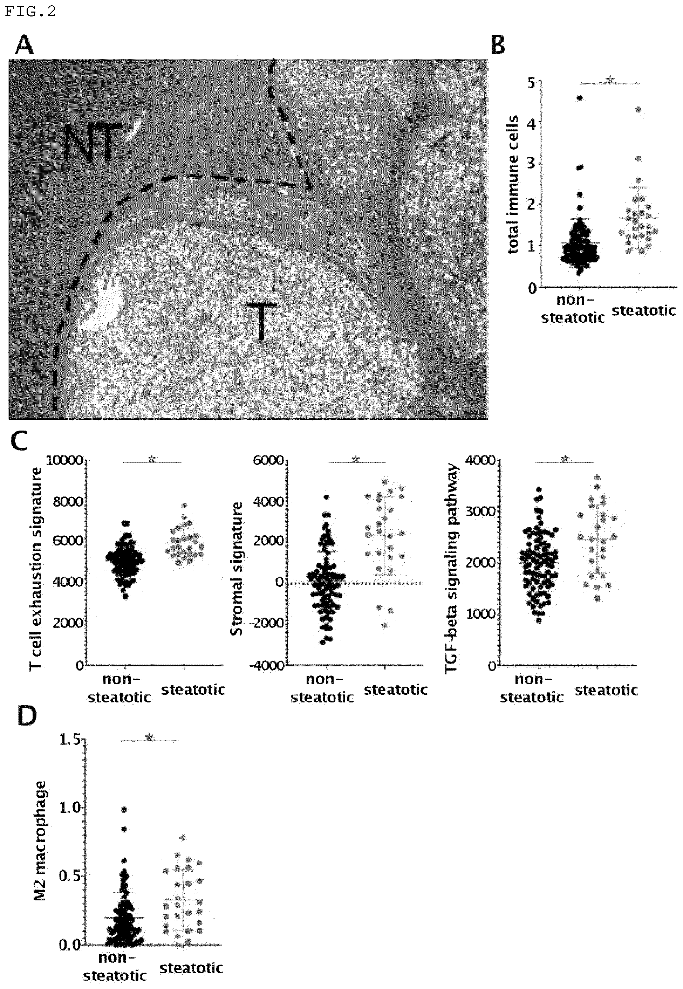

FIGS. 2 show the results of classification of steatotic hepatocellular carcinomas based on tumor immune microenvironments. FIG. 2A shows a representative image of steatotic hepatocellular carcinoma (T and NT stand for tumor and nontumor, respectively), FIG. 2B shows the results of comparison of CIBERSORT scores for total immune cells between steatotic hepatocellular carcinoma and nonsteatotic hepatocellular carcinoma, FIG. 2C shows the results of comparison of enrichment scores for a T cell exhaustion signature, a stromal signature, and a TGF-β signaling pathway between steatotic hepatocellular carcinoma and nonsteatotic hepatocellular carcinoma, and FIG. 2D shows the results of comparison of CIBERSORT scores for M2 macrophages between steatotic hepatocellular carcinoma and nonsteatotic hepatocellular carcinoma. In the figures, the "steatotic" means steatotic hepatocellular carcinoma, and the "non-steatotic" means nonsteatotic hepatocellular carcinoma. (Test Example 2)

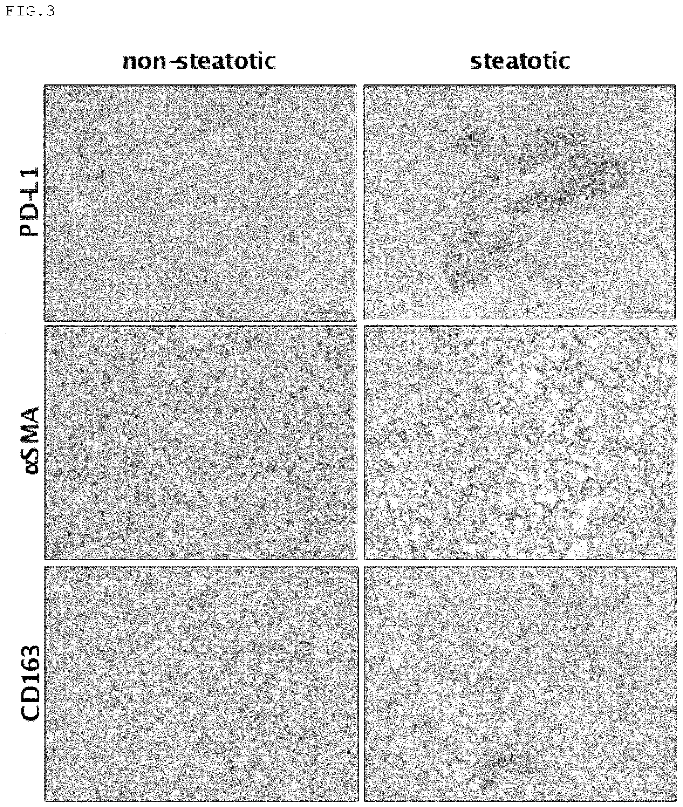

FIG. 3 shows the results of immunohistochemical analysis. Representative images of immunohistochemical staining for PD-L1, αSMA, and CD163 in steatotic hepatocellular carcinoma and nonsteatotic hepatocellular carcinoma are shown. In the figure, the "steatotic" means steatotic hepatocellular carcinoma, and the "non-steatotic" means nonsteatotic hepatocellular carcinoma. (Test Example 2)

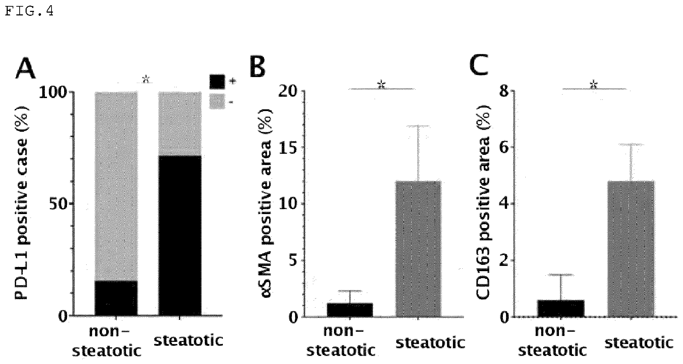

FIGS. 4 show the results of analysis of tumor immune microenvironments. FIG. 4A shows the results of comparison of the percentages of PD-L1 positive hepatocellular carcinoma between steatotic hepatocellular carcinoma and nonsteatotic hepatocellular carcinoma, FIG. 4B shows the results of comparison of the percentages of an αSMA positive area between steatotic hepatocellular carcinoma and nonsteatotic hepatocellular carcinoma, and FIG. 4C shows the results of comparison of the percentages of a CD163 positive area between steatotic hepatocellular carcinoma and nonsteatotic hepatocellular carcinoma. In the figures, the "steatotic" means steatotic hepatocellular carcinoma, and the "non-steatotic" means nonsteatotic hepatocellular carcinoma. (Test Example 2)

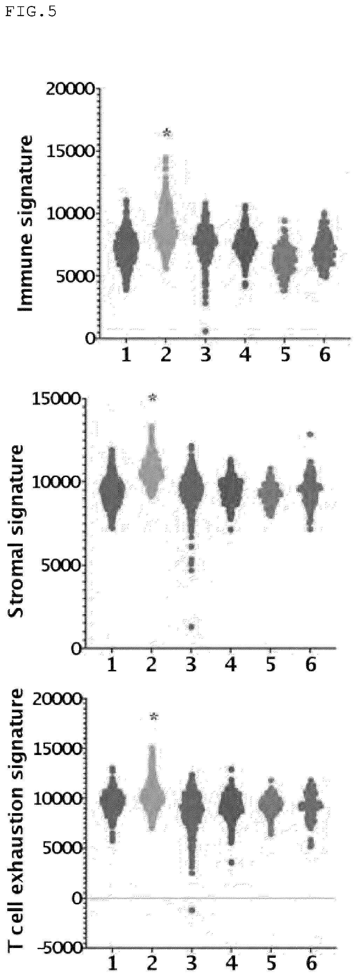

FIG. 5 shows the results of spatial transcriptomic analysis in steatotic hepatocellular carcinoma. Enrichment scores for the immune signature, stromal signature, and T cell exhaustion signature in each cluster are shown. (Test Example 2)

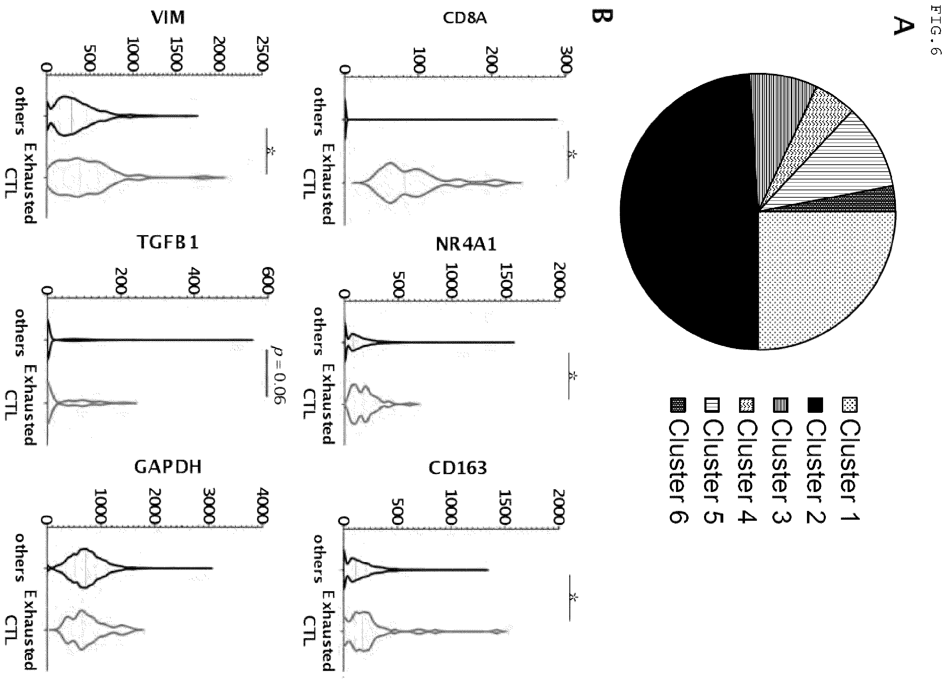

FIGS. 6 show the results of spatial transcriptomic analysis in steatotic hepatocellular carcinoma. FIG. 6A shows a pie chart showing the percentages of exhausted cytotoxic T-lymphocyte (CTL) spots defined as CD8A-positive and NR4A1-positive in each cluster, and FIG. 6B shows violin plots displaying the expression levels of T cell exhaustion markers CD8A and NR4A1, an M2 macrophage marker CD163, CAF markers VIM and TGFB1, and an internal reference GAPDH in the exhausted CTL spots and other spots. In the figures, the "Exhausted CTL" means exhausted cytotoxic T-lymphocyte spots, and the "others" means the other spots. (Test Example 2)

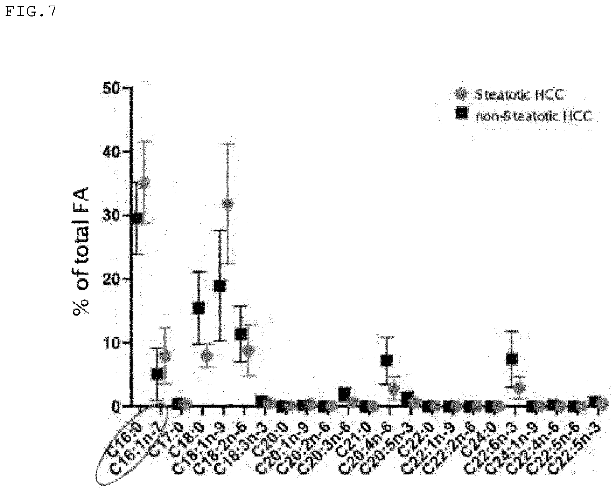

FIG. 7 shows the results of lipidomics-based total fatty acid profiling. In the figure, the "Steatotic HCC" means steatotic hepatocellular carcinoma, and the "non-Steatotic HCC" means nonsteatotic hepatocellular carcinoma. (Test Example 3)

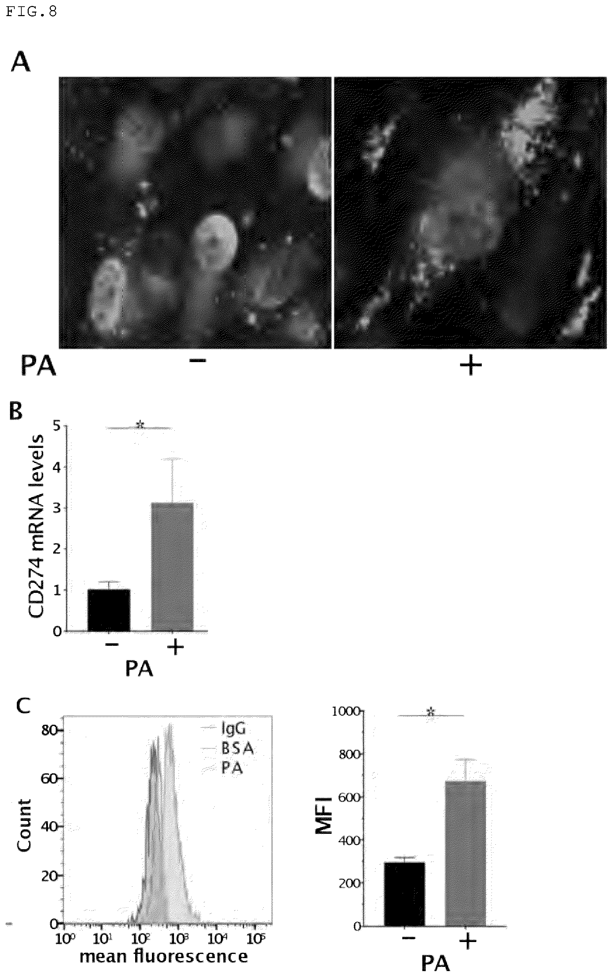

FIGS. 8 show the results of evaluation of the effect of lipid accumulation in tumor cells. FIG. 8A shows BODIPY-stained images in Hep3B cells 24 hours after bovine serum albumin (BSA) or palmitic acid (PA) supplementation, FIG. 8B shows relative mRNA levels of PD-L1 (CD274) in Hep3B cells 24 hours after BSA or PA supplementation, and FIG. 8C shows the results of flow cytometry analysis of PD-L1 (CD274) protein levels in Hep3B cells 24 hours after BSA or PA supplementation shown as a histogram (left) and mean fluorescence intensity (MFI) (right). In the figures, the "-" means bovine serum albumin supplementation, and the "+" means palmitic acid supplementation. (Test Example 3)

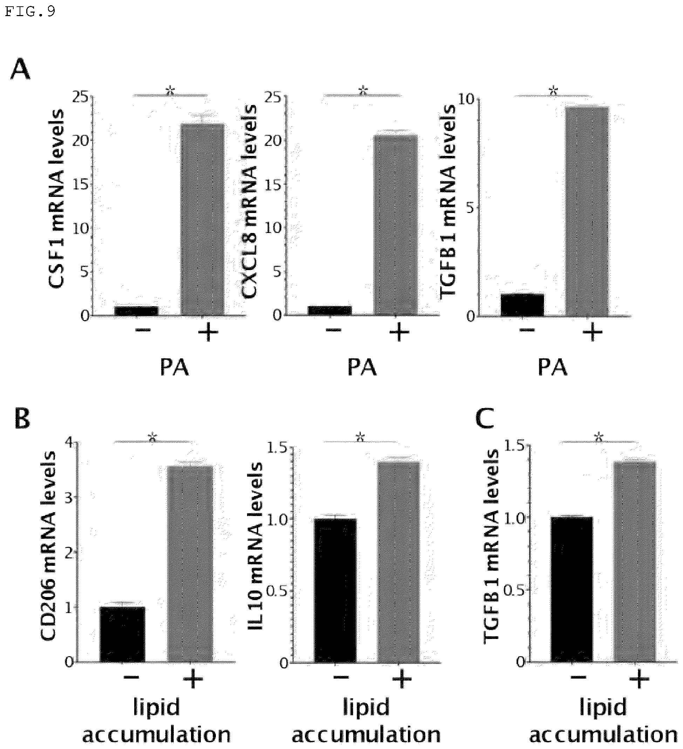

FIGS. 9 show the results of evaluation of the effect of lipid accumulation in tumor cells. FIG. 9A shows relative mRNA levels of CSF1, CXCL8, and TGFB1 in Hep3B cells 24 hours after BSA or PA supplementation, FIG. 9B shows relative mRNA levels of CD206 and IL10 in macrophages after 3 days of coculture with BSA- or PA-supplemented Hep3B cells, and FIG. 9C shows relative mRNA levels of TGFB1 in LX-2 cells after 3 days of coculture with BSA- or PA-supplemented Hep3B cells. In the figures, the "-" means bovine serum albumin supplementation (without lipid accumulation), and the "+" means palmitic acid supplementation (with lipid accumulation). (Test Example 3)

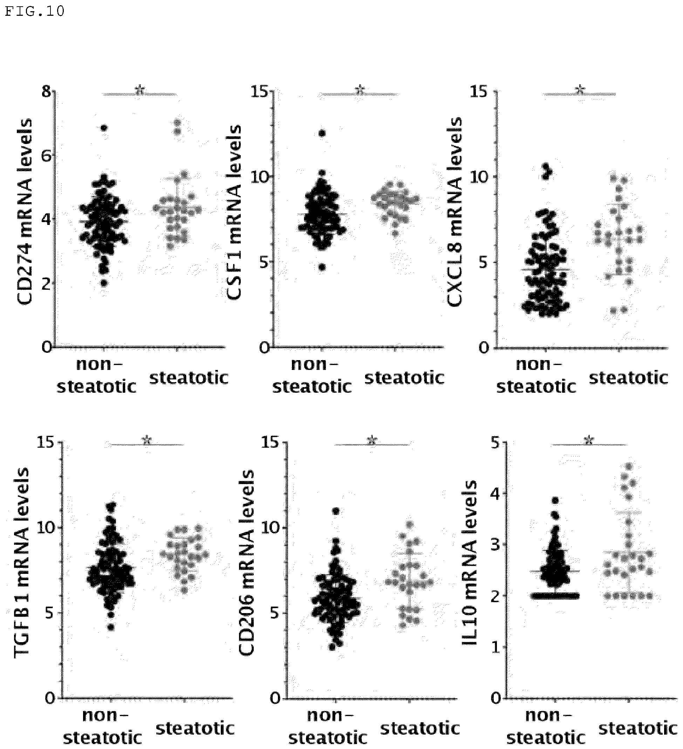

FIG. 10 shows the results of evaluation of the effect of lipid accumulation in tumor cells. Relative mRNA levels of PD-L1 (CD274), CSF1, CXCL8, TGFB1, CD206, and IL10 in steatotic hepatocellular carcinoma and nonsteatotic hepatocellular carcinoma are shown. In the figure, the "steatotic" means steatotic hepatocellular carcinoma, and the "nonsteatotic" means nonsteatotic hepatocellular carcinoma. (Test Example 3)

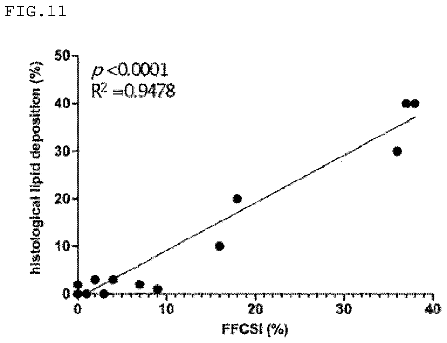

FIG. 11 is a correlation graph between $FF_{CSI}$ and histological lipid deposition in 20 surgically resected hepatocellular carcinoma samples. (Example 2)

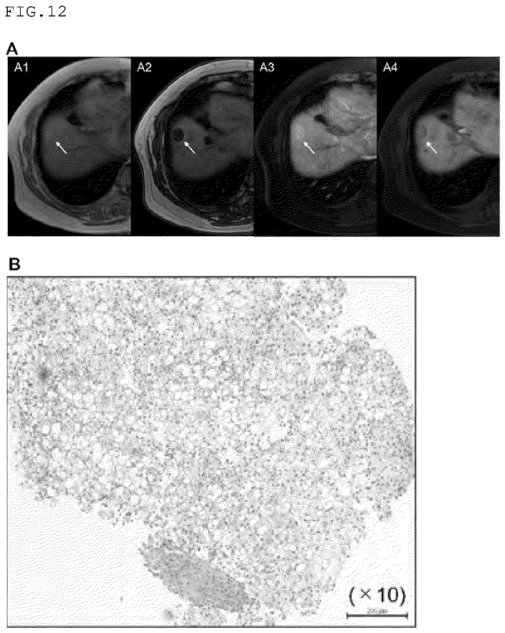

FIGS. 12 show identification of steatotic hepatocellular carcinoma by MRI. FIG. 12A1 shows an in-phase T1-weighted gradient-echo MR image (showing a well-defined hyperintense mass just below the diaphragm aspect of hepatic segment VIII (arrow)), FIG. 12A2 shows an opposed-phase T1-weighted gradient-echo MR image corresponding to A1 (showing a drop in the signal intensity of the tumor (arrow)), FIG. 12A3 shows a hepatic arterial phase Gd-EOB-DTPA-enhanced MR image (showing arterial enhancement of the tumor (arrow)), FIG. 12A4 shows 20-min hepatobiliary phase Gd-EOB-DTPA-enhanced MR image (showing a drop in the signal intensity of the tumor (arrow)), and FIG. 12B shows a hematoxylin-eosin image of the tumor biopsy specimen. In the figure, the bar represents 200 $\mu$m. (Example 3)

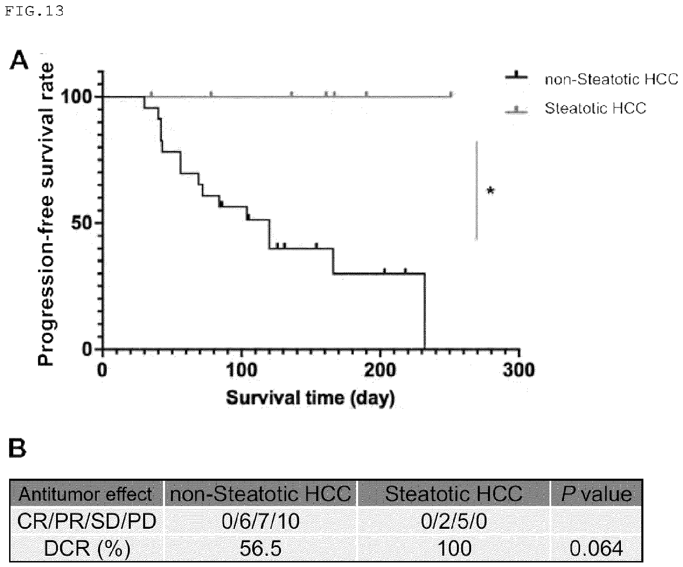

FIGS. 13 show the results of evaluation of therapeutic response of steatotic hepatocellular carcinoma to immunotherapy. FIG. 13A shows the results of Kaplan-Meier analysis of the progression-free survival rate of patients stratified by the presence or absence of steatosis in hepatocellular carcinoma, and FIG. 13B shows the results of the disease control rate (DCR) of patients stratified by the presence or absence of steatosis. In the figures, the "Steatotic HCC" means steatotic hepatocellular carcinoma, and the "non-Steatotic HCC" means nonsteatotic hepatocellular carcinoma. (Example 3)

Description of Embodiments

The present invention relates to a method of testing susceptibility to an immune checkpoint inhibitor, an apparatus for testing susceptibility to an immune checkpoint inhibitor, and a program for testing susceptibility to an immune checkpoint inhibitor.

The immune checkpoint inhibitor conceptually reinvigorates exhausted effector T cells, and is more effective in cancer with high tumor-infiltrated cytotoxic T cell and PD-L1 expression (JAMA Oncol. 2019 Aug 1; 5(8): 1195-1204.). The immune checkpoint inhibitor to which the test method of the present invention can evaluate susceptibility is not particularly limited, but may be, for example, at least one kind of an antibody directed against an immune checkpoint molecule, such as an anti-PD-1 antibody, an anti-PD-L1 antibody, an anti-PD-L2 antibody, an anti-CTLA-4 antibody, an anti-TIM-3 antibody, an anti-LAG-3 antibody, or an anti-TIGIT antibody, or an antibody directed against a ligand thereof. The immune checkpoint inhibitor is preferably, for example, an anti-PD-L1 antibody or an anti-PD-1 antibody, and is more preferably, for example, an anti-PD-L1 antibody.

The test method of the present invention also enables evaluation of susceptibility to combined therapy using the immune checkpoint inhibitor and any other drug. Examples of the other drug include an angiogenesis inhibitor, an anticancer antibiotic, and a hormone therapy agent. The angiogenesis inhibitor is not particularly limited, but may be, for example, a drug formulation containing at least one kind of an antibody directed against a vascular endothelial growth factor, such as an anti-VEGF antibody or an anti-VEGFR2 antibody, or an antibody directed against a receptor thereof. The other drug is preferably, for example, an angiogenesis inhibitor, and is more preferably, for example, an anti-VEGF antibody.

The immune checkpoint inhibitor of the present invention is preferably an immune checkpoint inhibitor used in combination with the other drug, and is more preferably an immune checkpoint inhibitor used in combination with an angiogenesis inhibitor. The immune checkpoint inhibitor is still more preferably an anti-PD-L1 antibody or an anti-PD-1 antibody used in combination with an angiogenesis inhibitor, and is most preferably an anti-PD-L1 antibody used in combination with an anti-VEGF antibody.

In the present invention, the "antibody" includes a polyclonal antibody, a monoclonal antibody, an antigen binding fragment of the antibody, a chimeric antibody including the antigen binding fragment, a recombinant antibody, and derivatives thereof.

The liver cancer serving as a target of the test method of the present invention includes metastatic liver cancer and primary liver cancer, and is preferably primary liver cancer, such as hepatocellular carcinoma or cholangiocellular carcinoma, and is more preferably hepatocellular carcinoma.

The susceptibility to the immune checkpoint inhibitor may also be restated as reactivity to the immune checkpoint inhibitor. Although cancer immunotherapy with the immune checkpoint inhibitor is used for many patients with liver cancer, not all of the patients achieve the same efficacy, and there are patients having high susceptibility and patients having low susceptibility. In addition, some patients become resistant thereto even after therapeutic efficacy is recognized. The susceptibility to the immune checkpoint inhibitor may be tested before the start of treatment or during treatment. When the susceptibility is revealed to be high by the test, satisfactory anticancer efficacy can be obtained by starting or continuing the administration of the immune checkpoint inhibitor. When the susceptibility is revealed to be low by the test, the administration of the other drug or any other treatment method may be chosen. Thus, an appropriate treatment method for a patient is provided, and treatment can be continued without a reduction in functional hepatic reserve.

The test method of the present invention uses a fat fraction as an indicator for the susceptibility to the immune checkpoint inhibitor, and is characterized by comprising a step of measuring a fat fraction in a liver cancer tissue. A measurement method for the fat fraction in the liver cancer tissue is not particularly limited. The fat fraction of the present invention is preferably calculated from an image obtained from the liver cancer tissue or a signal for displaying the image obtained from the liver cancer tissue. The image includes medical images, such as an MRI image, a CT image, an ultrasonic image, a stained image of tissue biopsy, and a histopathological image. In the present invention, the "MRI image" refers to an image obtained by magnetic resonance imaging (MRI), and is preferably a chemical-shift imaging image. In the present invention, the "chemical-shift imaging image" refers to an image obtained by chemical-shift imaging. Any known method may be used as an obtainment method for the image from the liver cancer tissue or the signal for displaying the image, and examples thereof include abdominal MRI, abdominal computed tomography (CT), abdominal echo, and HE staining of a liver biopsy tissue section. The "liver cancer tissue" in the present invention is not particularly limited as long as the tissue is a tumor tissue in the liver and may serve as a test target for the susceptibility to the immune checkpoint inhibitor. The tissue is preferably a tumor tissue in the liver of a subject that is affected by liver cancer or is suspected of being affected by liver cancer. The subject is not particularly limited, but is preferably a human or a pet animal, and is more preferably a human. The term "cancer tissue" or "tumor tissue" refers to a tissue including at least one tumor cell, and may include a connective tissue or blood vessel that supports the tumor.

The fat fraction in the present invention is not particularly limited as long as the fat fraction is an indicator representing the percentage of fat in the liver cancer tissue. The fat fraction may be, for example, the percentage of fat in the entire liver cancer tissue, or may be the percentage of fat measured, calculated, or estimated in a two-dimensional region or three-dimensional region in part of the liver cancer tissue. The fat fraction may be the percentage of the number of cells each having a lipid droplet in the number of cells in the liver cancer tissue (the ratio of cells each having a lipid droplet present therein, which is hereinafter also referred to as "steatosis rate"). In addition, the fat fraction may be the area or volume percentage of the lipid droplets obtained from the image. In addition, the fat fraction may be the percentage of a fat component in the liver cancer tissue. In addition, the fat fraction may be the percentage of fat calculated from the signal for displaying the image. In addition, the fat fraction may be a steatosis rate calculated from the image obtained from the liver cancer tissue, or a fat fraction calculated from the signal for displaying the image obtained from the liver cancer tissue. More specific examples of the fat fraction may include a steatosis rate calculated from a stained image of tissue biopsy, and a fat fraction calculated from the following expression by resolving a net MR signal intensity of MRI into a fat signal intensity and a water signal intensity: fat signal intensity/(fat signal intensity + water signal intensity).

A method to obtain the image for measuring the fat fraction in the test method of the present invention is not particularly limited, and may be an invasive method or a noninvasive method, but is preferably a noninvasive method because most patients with unresectable advanced liver cancer serving as immunotherapy targets undergo diagnosis and treatment without undergoing tumor biopsy. An example of the invasive method is tissue biopsy. Specifically, quantification can be performed by using the ratio of cells each having a large lipid droplet present therein in a stained image of liver cancer tissue biopsy. The noninvasive method is not particularly limited, and for example, MRI, CT, or an ultrasonic wave may be used. From the viewpoint of detection accuracy, MRI is more preferred.

Any existing method, for example, proton nuclear magnetic resonance spectroscopy ($^1$H-MRS), a 3-point Dixon (DIXON) method, a multi-echo gradient-echo (MEGE) method, chemical-shift imaging, or frequency-selective imaging may be applied as the measurement method for the fat fraction using MRI (Magn Reson Med Sci. 2011; 10(1): 41-8; Radiographics. 2009 Jan-Feb; 29(1): 231-60.). The measurement method for the fat fraction is preferably, for example, a method using chemical-shift imaging (CSI). The fat fraction may be more preferably calculated by the following equation (1) as a fat fraction measured by CSI ($FF_{CSI}$) based on the intensities of a water signal and a fat signal each resolved from an MR signal, and may be more specifically calculated from the percentage of a fat signal intensity in the total of the fat signal intensity and a water signal intensity.

$$FF_{CSI}\ (\%)\ =\ (S_{fat})/(S_{water}\ +\ S_{fat})\ \times 100\ \cdots\ (1)$$

In the equation (1), "$S_{fat}$" represents a fat signal intensity, and "$S_{water}$" represents a water signal intensity. The equation (1) may be converted to the following equation (2). In the equation (2), the fat fraction may be calculated from a signal intensity in a region of interest (ROI) common to an in-phase image and an opposed-phase image at a frequency of an MR signal obtained from a proton of a water molecule and a proton of a methylene group of a fat molecule. Specifically, on a tumor tissue in an MRI image, the region of interest is set to preferably the entirety or part of the tumor tissue in the MRI image, more preferably the entirety of the tumor tissue in the MRI image, and an average signal intensity in the region of interest in the in-phase image and an average signal intensity in the region of interest in the opposed-phase image may be applied to the equation (2) as IP and OP, respectively. The part of the tumor tissue is not particularly limited, but may be, for example, a part having an area of from 1 mm$^2$ to 10,000 mm$^2$, preferably from 20 mm$^2$ to 1,000 mm$^2$ in the contour of the tumor tissue. When the region of interest is set, the tumor tissue may be specified by using a contrast-enhanced MRI image. The contrast used for specifying the tumor tissue is not particularly limited as long as the contrast can be utilized for detection of liver cancer, and examples thereof may include gadoxetate sodium (Gd-EOB-DTPA) and super paramagnetic iron oxide (SPIO).

$$\mathrm{FF_{CSI}} \ (\%) \ = \ (\mathrm{IP-OP})/(2\times\mathrm{IP}) \ \times 100 \ \cdots \ (2)$$

In the equation (2), "IP" represents an in-phase signal intensity represented by the following equation (3), and "OP" represents an opposed-phase signal intensity represented by the following equation (4) (Radiographics. 2009 Jan-Feb; 29(1): 231-60.).

$$\mathrm{IP} = \left| \mathrm{S_{water}} \ + \ \mathrm{S_{fat}} \right| \ \cdots \ (3)$$

In the equation (3), "$\mathrm{S_{water}}$" represents a water signal intensity, and "$\mathrm{S_{fat}}$" represents a fat signal intensity.

$$\mathrm{OP} = \left| \mathrm{S_{water}} \ - \ \mathrm{S_{fat}} \right| \ \cdots \ (4)$$

In the equation (4), "$\mathrm{S_{water}}$" represents a water signal intensity, and "$\mathrm{S_{fat}}$" represents a fat signal intensity.

The test method of the present invention may further comprise a step of comparing the fat fraction with a reference value. In the test method of the present invention, a susceptibility level to the immune checkpoint inhibitor can be determined by comparing the fat fraction having been measured with a preset reference value. Examples of the susceptibility level include levels of having high susceptibility, having susceptibility, having low susceptibility, having resistance, and having moderate susceptibility. The susceptibility level may also be numerically represented in stages as, for example, a susceptibility level of 1 and a susceptibility level of 2. For example, when the fat fraction having been calculated is equal to or higher than the preset reference value, the susceptibility level can be determined as a level of having high susceptibility to the immune checkpoint inhibitor or having susceptibility thereto. When the fat fraction having been calculated is less than the preset reference value, the susceptibility level can be determined as a level of having low susceptibility to the immune checkpoint inhibitor, or having high resistance or having resistance thereto.

The reference value by which the susceptibility level is determined as a level of having high susceptibility to the immune checkpoint inhibitor or having susceptibility thereto may be, for example, a value selected from the range of 1% or more and 40% or less, for example, 1%, 2%, 3%, 4%, 5%, 6%, 7%, 8%, 9%, 10%, 11%, 12%, 13%, 14%, 15%, 17%, 20%, 25%, 30%, or 40%. The reference value may be, for example, a value selected from the range of preferably 3% or more and 20% or less, more preferably 5% or more and 15% or less, still more preferably 5% or more and 10% or less. The reference value by which the susceptibility level is determined as a level of having low susceptibility to the immune checkpoint inhibitor, or having high resistance or having resistance thereto may be, for example, a value selected from the range of 1% or more and 40% or less, for example, 40%, 30%, 25%, 20%, 17%, 15%, 14%, 13%, 12%, 11%, 10%, 9%, 8%, 7%, 6%, 5%, 4%, 3%, 2%, or 1%. The reference value may be, for example, a value selected from the range of preferably 3% or more and 20% or less, more preferably 5% or more and 15% or less, still more preferably 5% or more and 10% or less.

The reference value for the fat fraction may be set for every measurement method for the fat fraction. The susceptibility level to the immune checkpoint inhibitor (a level of having high susceptibility, having low susceptibility, having resistance, having moderate susceptibility, or the like) may be determined by comparing the fat fraction with the reference value. Now, setting of a reference value for the steatosis rate (the percentage of the number of cells each having a lipid droplet in the number of cells in the liver cancer tissue (the ratio of cells each having a lipid droplet present therein)), and setting of a reference value for the fat fraction $\mathrm{FF_{CSI}}$ (%) measured by chemical-shift imaging are further described.

The reference value for the steatosis rate may be set as described below. The reference value by which the susceptibility level is determined as a level of having high susceptibility to the immune checkpoint inhibitor or having susceptibility thereto may be, for example, a value selected from the range of 1% or more and 40% or less, for example, 1%, 2%, 3%, 4%, 5%, 6%, 7%, 8%, 9%, 10%, 11%, 12%, 13%, 14%, 15%, 17%, 20%, 25%, 30%, or 40%. The reference value may be, for example, a value selected from the range of preferably 3% or more and 15% or less, more preferably 4% or more and 10% or less, still more preferably 5%. The reference value by which the susceptibility level is determined as a level of having low susceptibility to the immune checkpoint inhibitor, or having high resistance or having resistance thereto may be, for example, a value selected from the range of 1% or more and 40% or less, for example, 40%, 30%, 25%, 20%, 17%, 15%, 14%, 13%, 12%, 11%, 10%, 9%, 8%, 7%, 6%, 5%, 4%, 3%, 2%, or 1%. The reference value may be, for example, a value selected from the range of preferably 3% or more and 15% or less, more preferably 4% or more and 10% or less, still more preferably 5%.

The reference value for the fat fraction $FF_{CSI}$ (%) measured by chemical-shift imaging may be set as described below. The reference value by which the susceptibility level is determined as a level of having high susceptibility to the immune checkpoint inhibitor or having susceptibility thereto may be, for example, a value selected from the range of 3% or more and 40% or less, for example, 3%, 4%, 5%, 6%, 7%, 8%, 9%, 10%, 11%, 12%, 13%, 14%, 15%, 17%, 20%, 25%, 30%, or 40%. The reference value may be, for example, a value selected from the range of preferably 5% or more and 20% or less, more preferably 5% or more and 15% or less, still more preferably 8% or more and 12% or less, most preferably 10%. The reference value by which the susceptibility level is determined as a level of having low susceptibility to the immune checkpoint inhibitor, or having high resistance or having resistance thereto may be, for example, a value selected from the range of 3% or more and 40% or less, for example, 40%, 30%, 25%, 20%, 17%, 15%, 14%, 13%, 12%, 11%, 10%, 9%, 8%, 7%, 6%, 5%, 4%, or 3%. The reference value may be, for example, a value selected from the range of preferably 5% or more and 20% or less, more preferably 5% or more and 15% or less, still more preferably 8% or more and 12% or less, most preferably 10%. The $FF_{CSI}$ (%) has a strong positive correlation with the steatosis rate. Accordingly, the reference value for the fat fraction $FF_{CSI}$ (%) may be the same as the above-mentioned reference value for the steatosis rate.

While Examples described later by no means limit the scope of the present invention, the inventors of the present invention have found in Examples that steatotic hepatocellular carcinoma in which the ratio of cells each having a large lipid droplet present therein in the liver cancer tissue is equal to or higher than the reference value is characterized by an immune-exhausted tumor immune microenvironment with high PD-L1 expression, and have further found that the steatotic hepatocellular carcinoma has high susceptibility to immune checkpoint inhibitor therapy.

(Apparatus for testing Susceptibility to Immune Checkpoint Inhibitor)

The apparatus for testing susceptibility to an immune checkpoint inhibitor of the present invention comprises any one or more of the following configurations:

a signal detection unit configured to detect an MR signal of a liver cancer tissue;
a fat fraction calculation unit configured to calculate a fat fraction in the liver cancer tissue from the MR signal having been detected; and
an output unit configured to output a susceptibility level to the immune checkpoint inhibitor based on comparison between the fat fraction having been calculated and a reference value.

The signal detection unit detects an MR signal generated through a nuclear magnetic resonance phenomenon. The MR signal having been detected is transmitted to and received by the fat fraction calculation unit by any known data transmitting/receiving means as signal data. The fat fraction calculation unit calculates a fat fraction from the MR signal having been detected in the signal detection unit. A preferred calculation method includes resolving a net MR signal into a water signal and a fat signal, and calculating the fat fraction from a fat signal intensity with respect to the total of the fat signal intensity and a water signal intensity. The output unit outputs a susceptibility level to the immune checkpoint inhibitor according to a comparison result obtained by comparison between the fat fraction having been calculated in the fat fraction calculation unit and a preset reference value. The susceptibility level may be the presence or absence of susceptibility, or may be quantified or stratified as a susceptibility level according to the amount of the fat fraction. The test apparatus of the present invention may be a single apparatus, or may be an apparatus externally connected to any known MRI apparatus. When the test apparatus of the present invention is an apparatus externally connected to an MRI apparatus, the signal detection unit is incorporated in the MRI apparatus, and the fat fraction calculation unit and the output unit are each incorporated in a computer that is externally connected to the MRI apparatus and includes a CPU, a storage medium, and the like. The embodiments of the respective configurations according to the test apparatus of the present invention are the same as the embodiments described in the test method.

(Program for testing Susceptibility to Immune Checkpoint Inhibitor)

The program for testing susceptibility to an immune checkpoint inhibitor of the present invention causes a computer to execute any one or more of the following steps:

a step of inputting data on an MR signal of a liver cancer tissue;

a step of calculating a fat fraction in the liver cancer tissue from the data on the MR signal having been input; and
a step of outputting a susceptibility level based on comparison between the fat fraction having been calculated and a reference value.

In the step of inputting data on an MR signal, data on an MR signal generated through a nuclear magnetic resonance phenomenon is input. In the step of calculating a fat fraction, a fat fraction is calculated from the data on the MR signal having been input. A preferred calculation method includes resolving net MR signal data into water signal data and fat signal data, and calculating the fat fraction from a fat signal intensity with respect to the total of the fat signal intensity and a water signal intensity. In the step of outputting a susceptibility level, a susceptibility level to the immune checkpoint inhibitor is output according to a comparison result obtained by comparison between the fat fraction having been calculated and a preset reference value. The susceptibility to the immune checkpoint inhibitor can be tested by installing the test program of the present invention in any known MRI apparatus or an apparatus externally connected thereto. The embodiments of the respective configurations according to the test program of the present invention are the same as the embodiments described in the test method.

Examples

For better understanding of the present invention, the present invention is specifically described below by way of Examples, which by no means limit the scope of the present invention. The following test was consistent with the principles of the Declaration of Helsinki, and approved by the Institutional Review Board committee at Osaka University Hospital. The test was performed after all patients provided written informed consent.

1. Method

(DNA and RNA Extraction)

Genomic DNA and total RNA were extracted from tissue specimens by using the DNeasy Blood & Tissue Kit (QIAGEN, Venlo, Netherlands) and RNeasy Mini Kit (QIAGEN), respectively, by a previously reported procedure (Proc Natl Acad Sci USA. 2018; 115: E10417-E10426.). The integrity of the obtained DNA and RNA was confirmed by using a 2100 Bioanalyzer (Agilent Technologies, Santa Clara, CA, US).

(RNA Sequencing Analysis)

Total RNA was isolated from liver tissues by a previously reported procedure (Gastroenterology. 2010 Jun; 138(7): 2487-98, 2498. e1-7.). Library preparation was performed by using TruSeq Stranded mRNA Sample Prep Kit (Illumina, San Diego, CA, US) on Apollo Library Prep System (TaKaRa). Sequencing was performed on Illumina HiSeq 3000 platform in a 75-base single-end mode. Sequenced reads were mapped to the human reference genome sequence (hg19) by using TopHat v2.1.1. The normalization of counted data was performed by using a relative log expression (RLE) method or a trimmed mean of M values (TMM) method.

(Genome Sequencing Analysis)

After the library preparation, custom PCR primer sets for amplicon sequencing targeting the coding regions of 69 genes (Table 1), in which recurrent genetic alterations had been previously reported in hepatocellular carcinoma, and TERT promoter regions were designed by Ion AmpliSeq Designer. The genomic DNA extracted from 55 pairs of tumor and normal tissues was used for the library preparation. The following two different methods were used for the library preparation. One is Ion AmpliSeq Library Kit 2.0 (Thermo Fisher Scientific, Waltham, MA, US), and the other one is Ion AmpliSeq Kit for Chef DL8 (Thermo Fisher Scientific). In the former, multiplex PCR was performed by using 30 ng of DNA as a template. The amplified products were further ligated to barcode adapters (IonXpress Barcode Adapters; Thermo Fisher Scientific) for sample identification, and purified with Agencourt AMPure XP Reagent (Beckman Coulter, Brea, CA, US). In the latter, 10 ng of DNA was used as a template. Multiplex PCR, ligation to barcode adapters (IonCode Barcode Adapters; Thermo Fisher Scientific), and sample purification were automatically performed by Ion Chef system (Thermo Fisher Scientific). After emulsion PCR was performed by using Ion PI HI-Q Chef Kit (Thermo Fisher Scientific) with 14 to 16 samples per Ion PI chip v3, the libraries were sequenced. The libraries were sequenced by Ion Proton using Ion PI Hi-Q Sequencing 200 Kit. FASTQ files obtained from the sequencing were aligned to the human reference genome sequence (GRCh38) by using Burrows-Wheeler Alignment tool v0.7.12 (Bioinformatics. 2009 Jul 15; 25(14): 1754-60.). The alignments were converted from a Sequence Alignment Map (SAM) format to Binary Alignment Map (BAM) files by SAMtools v1.9 (Bioinformatics. 2009 Aug 15; 25(16): 2078-9.). Realignment and recalibration of base quality scores were performed by using Genome Analysis Toolkit version 4.1. In order to perform single nucleotide variant (SNV) analysis, pileup files were created from the BAM files by using SAMtools, and somatic mutations were detected by using VarScan2 v2.4 (Genome Res. 2012 Mar; 22(3): 568-76.). Out of tumor tissue-specific mutations having been filtered, one having a minor allele frequency of less than 0.05 and being regarded as significant (P<0.05) by Fisher's exact test was considered to be a mutation gene candidate. Variants having passed through the filter were annotated by using ANNOVAR (Nucleic Acids Res. 2010 Sep; 38(16): e164.), and compared with dbSNP (build 150) and COSMIC (ver. 90) databases. Information on functional prediction of non-synonymous mutations was obtained from SIFT and Polyphen databases. In order to detect copy number variations (CNVs), DeCON (Wellcome Open Res. 2016 Nov 25; 1: 20.) was used for comparison of copy numbers between BAM files of 55 normal tissue sample sets and one tumor sample.

Table 1

| | | |
|---|---|---|
| ACVR2A | CSMD1 | MDM2 |
| AHCTF1 | CTNNB1 | MDM4 |
| AKT1 | EEF1A1 | MET |
| AKT2 | ERRFI1 | MYC |
| ALB | FGF19 | NCOR1 |
| APC | FGFR1 | NF1 |
| APOB | FOXP1 | NFE2L2 |
| ARID1A | GNAS | NRAS |
| ARID1B | GPATCH4 | PIK3CA |
| ARID2 | HIST1H1C | PTEN |
| AXIN1 | HNF1A | RB1 |
| AZIN1 | IDH1 | RP1L1 |
| BAP1 | IDH2 | RPS6KA3 |
| BRD4 | IGF1R | SF3B1 |
| BRD7 | IL6ST | SMARC4 |
| CCND1 | JAK2 | SMARCA4 |
| CCNE1 | KEAP1 | STAT3 |
| CD274 | KMT2C | TERT |
| CDK4 | KMT2D | TP53 |
| CDK6 | KRAS | TSC1 |
| CDKN2A | LINC00290 | TSC2 |
| CREB3L3 | LRPIB | VEGFA |
| CREBBP | LZTR1 | WWOX2 |

(Bioinformatics Analysis)

Unsupervised hierarchical clustering, principal component analysis (PCA), detection of differentially expressed genes, and generally applicable gene-set enrichment (GAGE) pathway analysis focusing on Gene Ontology (GO) biological processes of RNA-seq data were performed by using iDEP92 (http://bioinformatics.sdstate.edu/idep92/). The immune class was determined by nearest template prediction (NTP) analysis using an immune subclass gene classifier (Gastroenterology. 2017 Sep; 153(3): 812-826.). Single-sample gene set enrichment analysis (ssGSEA) and NTP were performed by using Gene Pattern (https://cloud.genepattern.org/gp/pages/index.jsf). CIBERSORT analysis using RNA-seq data was performed in an absolute mode with an LM22 signature matrix on a website (https://cibersort. stanford.edu/).

(Immunohistochemistry)

A liver sample was embedded in a paraffin block and stained with hematoxylin-eosin according to a standard method. Hepatocellular carcinoma in which lipid droplets were present in more than 5% of its tumor cells was defined as steatotic hepatocellular carcinoma (steatotic HCC). Immunohistochemistry for CD163, $\alpha$SMA, and PD-L1 was performed in a patient subset from a cohort of 113 patients. Immunohistochemical staining was performed for a formalin-fixed paraffin-embedded (FFPE) tissue section having a thickness of 3 $\mu$m after heat-induced antigen retrieval with a microwave using 10 mM TRIS-EDTA (having a pH of 9.0 in the case of CD163 and having a pH of 6.0 in the cases of $\alpha$SMA and PD-L1). Primary antibodies used were an anti-CD163 antibody (Proteintech, Rosemont, IL, US), an anti-$\alpha$SMA antibody (Abcam, Cambridge, UK), and an anti-PD-L1 antibody (Abcam, clone 28-8). In the cases of CD163 and $\alpha$SMA, positively stained areas were quantified by using BZ-X700 (KEYENCE, Osaka, Japan), and were used for comparison analysis between steatotic hepatocellular carcinoma and nonsteatotic hepatocellular carcinoma. PD-L1 expression was evaluated for neoplastic hepatocellular carcinoma (neoplastic HCC) cells. As previously reported for hepatocellular carcinoma and other cancers (Hepatology. 2016 Dec; 64(6): 2038-2046.), the percentage of neoplastic cells each showing membranous staining was recorded, and tumors with at least 1% positive cells were classified as positive.

(Cell Line and Reagent)

A Hep3B human liver cancer cell line, a THP-1 human monocyte-derived cell line, and an LX-2 human hepatic stellate cell line were purchased from American Type Culture Collection (ATCC, Manassas, VA, US). Those cell lines were each cultured in a DMEM or RPMI-1640 medium supplemented with 10% fetal calf serum and an antibiotic. It was confirmed that all the cells were free of any pathogen and mycoplasma. Bovine serum albumin (BSA, fat free) was purchased from Wako Pure Chemical Industries, Ltd., and was used as a control for palmitic acid (PA). Palmitic acid was purchased from Sigma-Aldrich, and was dissolved in ethanol to prepare a stock solution, which was diluted with a cell growth medium for an assay. Phorbol 12-myristate 13-acetate (PMA) was purchased from Sigma-Aldrich, and was dissolved in DMSO to prepare a stock solution, which was diluted with a cell growth medium for an assay. PMA was used as a stimulating agent for differentiation of THP-1 cells into macrophages.

(Palmitic Acid Supplementation)

Hep3B cells were plated in a 6-well plate at a density of $1.0 \times 10^5$ cells/well one day before palmitic acid supplementation. The next day, the cells were supplemented with palmitic acid at a concentration of 400 $\mu$M for 24 hours.

(Coculture System for Lipid-accumulated Hep3B Cells and Macrophages or Fibroblasts)

THP-1 cells were plated in a 6-well plate at a density of $1.0 \times 10^5$ cells/well one day before PMA supplementation. The next day, the cells were induced to differentiate into macrophages by being supplemented with PMA at a concentration of 10 ng/ml for 24 hours. The next day, after a supernatant containing palmitic acid was removed, the cells were cocultured with lipid-accumulated Hep3B cells by a transwell assay for 72 hours. LX-2 cells were plated in a 6-well plate at a density of $1.0 \times 10^5$ cells/well one day before coculture. The next day, after a supernatant containing palmitic acid was removed, the cells were cocultured with lipid-accumulated Hep3B cells by a transwell assay for 72 hours.

(Realtime PCR)

Total RNA was extracted from cells by using RNeasy Mini Kit (Qiagen) according to manufacturer's instructions. RNA was reverse transcribed by using SuperScript VILO Master Mix (Thermo Fisher Scientific). qPCR was performed by using a QuantStudio 12K Flex RT-PCR system with TaqMan Gene Expression Assay probes (Thermo Fisher Scientific) in the same manner as previously reported (Gastroenterology. 2016 Aug; 151(2): 324-337. e12.).

(Flow Cytometry)

1,000,000 Cells were mixed with 0.25 ml of a phycoerythrin (PE)-labeled anti-CD274 antibody (0.5 mg/ml) (BioLegend, San Diego, CA, US), and incubated in the dark at room temperature for 15 minutes. After washing, the cells were analyzed by using a Becton Dickinson FACS Canto II flow cytometer (BD Pharmingen, San Diego, CA, US).

(Lipidomics-based Total Fatty Acid Profiling)

Fatty acids in the liver were measured by using a gas chromatography-flame ionization detector (GC-FID) as previously reported (Bio Protoc. 2020 May 5; 10(9): e3613.). Briefly, a frozen liver sample was weighed and pulverized with Auto Mill (Tokken, Inc.). Total lipids were extracted from tissue powder having been pulverized by a Bligh and Dyer method (Can J Biochem Physiol. 1959 Aug; 37(8): 911-7.). As an internal standard, methyl tricosanoate (C23:0) was added to the lipids having been collected. After that, the fatty acids were methylated by using Fatty Acid Methylation Kit (Nacalai Tesque, Inc.). The fatty acid methyl esters were measured with GC-2010 Plus system (Shimadzu Corporation) having a flame ionization detector (FID) installed therein. FAMEWAX (30 m, inner diameter: 0.25 mm, 0.25 $\mu$m, Restek Corporation) was used as a separation capillary column. The flow rate of a carrier gas was set to a linear velocity of 45 cm/sec. An oven temperature was first set to 140°C, increased up to 200°C at a rate of 11°C/min, increased up to 225°C at a rate of 3°C/min, finally increased up to 240°C at a rate of 20°C/min, and maintained at this temperature for 5 minutes. An injection volume in a split injection mode was 2 $\mu$L. The respective fatty acid methyl esters were identified and quantified by using a mixture of fatty acid methyl ester standards (Supelco 37 Component FAME Mix and 2-(diisopropylamino)ethyl methacrylate (DPA) (n-3) (Sigma-Aldrich); DPA (n-6) (NU-CHEK PREP, INC., Elysian, MN, US); DTA (n-6) (Cayman)) for calibration. The values of the respective fatty acids were normalized with the value of C23:0.

(Statistical Analysis)

The data was expressed as a mean value $\pm$ standard deviation. Statistical analysis was performed by using a Mann-Whitney U test to assess a difference between unpaired groups. One-way analysis of variance (ANOVA) followed by a Kruskal-Wallis test was performed for multiple comparison. Fisher's exact test was used to analyze categorical data. A correlation was assessed by using the Pearson product-moment correlation coefficient. A Kaplan-Meier method and a log-rank test were used to analyze differences in overall survival rate (OS) or progression-free survival rate (PFS). Univariate logistic regression analysis and multivariate logistic regression analysis were used to analyze factors associated with immune class in nonviral hepatocellular carcinoma. Odds ratios and 95% confidence intervals (CI) are shown. The "p value<0.05" indicates statistical significance unless otherwise indicated. Prism version 8.4.2 for Mac (GraphPad Prism, RRID: SCR_002798, San Diego, CA, US) and SPSS software version 24 (IBM Corporation, Armonk, NY, US) were used for the analysis.

2. Test Example

(Test Example 1) Stratification through Multiomics Profiling

113 Patients who underwent curative hepatic resection for nonviral hepatocellular carcinoma between 2005 and 2018 at The Cancer Institute Hospital of Japanese Foundation for Cancer Research and Kagoshima University Hospital were enrolled. Those patients were confirmed for the absence of chronic liver disease, including viral hepatitis and autoimmune hepatitis. Snap-frozen hepatocellular carcinoma tissues were used for RNA and DNA sequencing, and formalin-fixed paraffin-embedded (FFPE) hepatocellular carcinoma tissues and surrounding liver tissues were used for histological analysis. Tumor RNA sequencing was performed in order to understand the molecular abnormalities of nonviral hepatocellular carcinoma. Through unsupervised hierarchical clustering analysis of transcriptomics, 113 patients with nonviral hepatocellular carcinoma were classified into three molecular classes (Class I, Class II, and Class III). "n" was 36 in Class I, "n" was 46 in Class II, and "n" was 31 in Class III. Patients with Class I had the poorest prognosis, whereas patients with Class III had the best prognosis (p<0.05). Next, cancer genome sequencing was conducted on 55 patients with nonviral hepatocellular carcinoma by using panels targeted at 69 genes in which genetic abnormalities were previously reported in hepatocellular carcinoma for a plurality of patients (Cell. 2017 Jun 15; 169(7): 1327-1341. e23.). Somatic mutations were detected in 50 of 55 samples and frequently observed in the TERT promoter region (58%), CTNNB1 (36%), and TP53 (18%) (FIG. 1C). Integrative analysis revealed close associations of Class I with the TP53 mutation (p<0.05) and Class III with the CTNNB1 mutation (p<0.05). Patients with nonviral hepatocellular carcinoma with TP53 mutations had the poorest prognosis, whereas patients with nonviral HCC with CTNNB1 mutations had the best prognosis (p<0.05 for TP53 vs. CTNNB1).

(Test Example 2) Classification of Nonviral Hepatocellular Carcinoma based on Tumor Immune Microenvironment (TIME)

In order to classify nonviral hepatocellular carcinomas based on tumor immune microenvironments, nearest template prediction (NTP) analysis of tumor transcriptomes targeted at 113 patients of Test Example 1 was performed. As a result of the NTP analysis, the immune class that was reported to be a subtype characterized by strong intratumoral immune cell infiltration in hepatocellular carcinoma (Gastroenterology. 2017 Sep; 153(3): 812-826.) was identified. Forty-three out of 113 tumors were categorized into the immune class and showed significantly higher levels of estimated total intratumor immune cells and cytotoxic T-lymphocytes (CTLs) by CIBERSORT analysis (FIG. 1A). In FIG. 1A, * represents p<0.05 for the immune class vs. the other class. The immune class showed the enrichment of tumors in Class II and significantly less frequent mutations of CTNNB1 (FIG. 1B and FIG. 1C). In FIG. 1B and FIG. 1C, * represents p<0.05 for the immune class vs. the other class. Consistently, nonviral hepatocellular carcinomas with CTNNB1 mutation showed significantly lower levels of intratumoral immune cell infiltration (FIG. 1D), which is in agreement with previous reports (Clin Cancer Res. 2019 Apr 1; 25(7): 2021-2023.). In FIG. 1D, * represents p<0.05 for hepatocellular carcinoma with CTNNB1 mutation vs. hepatocellular carcinoma without CTNNB1 mutation.

The clinicopathological factors that characterize the immune class were searched, and it was found that steatosis in hepatocellular carcinoma was strongly associated with the immune class. Indeed, it was shown that steatotic hepatocellular carcinoma, which accounts for 23% of nonviral hepatocellular carcinoma (FIG. 2A), contained significantly higher levels of total intratumor immune cells than nonsteatotic hepatocellular carcinoma (FIG. 2B). In FIG. 2B, * represents p<0.05 for nonsteatotic hepatocellular carcinoma vs. steatotic hepatocellular carcinoma. Interestingly, ssGSEA and pathway analysis showed significantly higher levels of the T cell exhaustion signature and stromal signature together with activation of TGF-β signaling in steatotic hepatocellular carcinoma (FIG. 2C), all of which were characteristics of exhaustion of tumor immunity (Nat Med. 2010 Oct; 16(10): 1147-51.; Nat Commun 4, 2612 (2013).; Cancer Cell. 2018 Apr 9; 33(4): 547-562.: Immunity. 2014 Sep 18; 41(3): 427-439.). In FIG. 2C, * represents p<0.05 for nonsteatotic hepatocellular carcinoma vs. steatotic hepatocellular carcinoma. Consistently, steatotic hepatocellular carcinoma showed the upregulation of a variety of immune checkpoints and transcription factors involved in T cell exhaustion and markers of cancer-associated fibroblasts (CAFs). Further, the CIBERSORT analysis showed the substantial infiltration of M2 macrophages in steatotic hepatocellular carcinoma, together with the upregulation of cytokines and chemokines involved in M2 polarization of macrophages and immunosuppression (FIG. 2D). In FIG. 2D, * represents p<0.05 for nonsteatotic hepatocellular carcinoma vs. steatotic hepatocellular carcinoma. The immunohistochemical analysis confirmed the upregulation of PD-L1 in tumor cells and substantial infiltration of CAFs and M2 macrophages in nonviral hepatocellular carcinoma (FIG. 3 and FIG. 4A to FIG. 4C). In FIG. 4A to FIG. 4C, "n" is from 5 to 13 in each group, and * represents p<0.05 for nonsteatotic hepatocellular carcinoma vs. steatotic hepatocellular carcinoma. Overall, steatotic hepatocellular carcinoma presented an immune-enriched but immune-exhausted tumor immune microenvironment characterized by T cell exhaustion, infiltration of M2 macrophages and CAFs, high PD-L1 expression, and TGF-β signaling activation.

In order to further characterize the immune-exhausted tumor immune microenvironment in steatotic hepatocellular carcinoma, the topography of M2 macrophages, CAFs, and CTLs in steatotic hepatocellular carcinoma was examined by spatial gene expression analysis on Visium platform. The sample of steatotic hepatocellular carcinoma was embedded in O.C.T. Compound (TissueTek, Sakura) in a 10 mm×10 mm cryomold at -80°C and sectioned at a thickness of 10 μm (Leica CM3050 S). Libraries for Visium were prepared according to instructions (Visium Spatial Gene Expression User Guide; CG000239_VisiumSpatialGeneExpression_UserGuide_Rev_A.pdf). Tissue was permeabilized for 3 minutes, which was identified as the optimal time in tissue optimization time course experiments. Libraries were sequenced on NovaSeq 6000 System (Illumina) by using NovaSeq S4 Reagent Kit (200 cycles, catalog number: 20027466, Illumina) at a sufficient sequencing depth. Raw FASTQ files and histology images were processed by using Space Ranger software v1.2.1 (https://support.10xgenomics.com/spatial-gene-expression/software/pipelines/latest/installation). In order to visualize spatial expression using histological images, the raw Visium files for each sample were read into Loupe Browser software v4.0.0 (https://support.10xgenomics.com/spatial-gene-expression/software/downloads/latest). As a result, mean base sequence read counts of 288,702 were obtained, and median genes of 3,300 per spot were identified.

The tumor section was divided into 1,768 spots, and transcriptomic data were obtained from each spot. Graph-based clustering divided all the spots into six clusters, with high expression of immune cell populations in cluster 2 (FIG. 5). ssGSEA showed that the stromal signature and T cell exhaustion signature were enhanced in cluster 2 (FIG. 5). In FIG. 5, * represents p<0.05 for cluster 2 vs. the other cluster. When CD8A and NR4A1 double-positive spots were extracted as spots containing exhausted CTLs, it was found that almost half of those spots were included in cluster 2 (FIG. 6A). Next, transcriptomic profiles are compared between spots with or without exhausted CTLs. As a result, there was found increased expression of the M2 macrophage marker CD163 and the CAF marker VIM in addition to elevated TGFB1 levels in the spots with exhausted CTLs (FIG. 6B). In FIG. 6B, * represents p<0.05 for spot with exhausted CTL2 vs. the other spot. Those results suggested that M2 macrophages and CAFs produce TGF-β and promote exhaustion of surrounding CTLs in close proximity, forming an immune-exhausted TIME in steatotic hepatocellular carcinoma.

(Test Example 3) Evaluation of Effect of Lipid Accumulation in Tumor Cells

The mechanistic link between the intratumor state and the immune-exhausted TIME in steatotic hepatocellular carcinoma was investigated. For this purpose, first, lipidomics-based total fatty acid profiling was performed, and it was found that palmitic acid (C16:0) and palmitoleic acid (C16:1n-7) levels were higher in steatotic hepatocellular carcinoma than in nonsteatotic hepatocellular carcinoma (FIG. 7). In view of the foregoing, the effect of accumulation of palmitic acid on steatotic hepatocellular carcinoma cells in vitro was investigated. Palmitic acid supplementation in Hep3B cells induced lipid accumulation (FIG. 8A), upregulated PD-L1 (CD274) expression at the mRNA and surface protein levels (FIG. 8B and FIG. 8C), and further, upregulated expression levels of CSF1, CXCL8, and TGFB1 (FIG. 9A). In view of the foregoing, the inventors further investigated the effect of accumulation of palmitic acid in tumor cells on surrounding macrophages and fibroblasts in vitro. Palmitic acid-treated Hep3B cells upregulated the expression levels of CD206 and IL10 in a cocultured human macrophage cell line (FIG. 9B) and the expression level of TGFB1 in a cocultured human hepatic stellate cell line (FIG. 9C). In FIG. 8B, FIG. 8C, and FIG. 9A to FIG. 9C, "n" is 3 in each group, and * represents $p<0.05$ for palmitic acid-supplemented group vs. BSA-supplemented (control) group. Upregulation of those immunosuppressive cytokines and chemokines was also observed in steatotic hepatocellular carcinoma in vivo (FIG. 10). In FIG. 10, * represents $p<0.05$ for nonsteatotic hepatocellular carcinoma vs. steatotic hepatocellular carcinoma. Those data suggested that the accumulation of palmitic acid in tumor cells promotes immunosuppression, which contributes to the development of an immune-exhausted TIME in steatotic hepatocellular carcinoma.

In Test Examples described above, the inventors of the present invention stratified nonviral hepatocellular carcinomas according to prognosis or the tumor immune microenvironments (TIMEs) by multiomics profiling, and identified the link between an intratumoral fat tissue and an immune-exhausted immunotherapy-susceptible TIME in hepatocellular carcinoma.

3. Examples

(Example 1) Measurement of Fat Fraction using Histopathological Image

Surgically resected hepatocellular carcinoma tissue pieces obtained from 20 patients with hepatocellular carcinoma (HCC) were each stained with hematoxylin-eosin (HE) and measured for the number of lipid droplet-containing cells, and the percentage of the number of lipid droplet-containing cells in the number of cells in the hepatocellular carcinoma tissue was calculated as a fat fraction (a steatosis rate, histological lipid deposition) (FIG. 11).

(Example 2) Measurement of Fat Fraction in Hepatocellular Carcinoma by MRI

20 Patients with HCC described in Example 1 underwent abdominal MRI including chemical-shift imaging (CSI) before extirpative surgery of their cancer tissues. The signal intensity of the largest tumor for each patient was acquired by drawing regions of interest (ROI) from both in-phase and opposed-phase images at the same level. The drawing of the ROI was performed by manually drawing the contour of the tumor by using Horos (trademark) software (Nimble Co., LLC, d/b/a Purview). The fat fraction measured by CSI ($FF_{CSI}$) was calculated by the following equation (2). It was determined that the hepatocellular carcinomas of 5 patients each having a fat fraction of 10% or more had susceptibility to the immune checkpoint inhibitor (FIG. 11).

$$FF_{CSI} \; (\%) \; = \; (IP-OP)/(2 \times IP) \; \times 100 \; \cdots \; (2)$$

In the equation (2), "IP" represents an average signal intensity in the region of interest in the in-phase image, and "OP" represents an average signal intensity in the region of interest in the opposed-phase image (Radiographics. 2009 Jan-Feb; 29(1): 231-60.).

A strong positive correlation was observed between the steatosis rate based on the histopathological image and $FF_{CSI}$ measured with the chemical-shift imaging image (FIG. 11). Thus, it was recognized that MRI was a reliable tool to identify steatotic hepatocellular carcinoma.

(Example 3) Identification of Steatotic Hepatocellular Carcinoma by MRI and Evaluation of Therapeutic Response to Immunotherapy

Combined immunotherapy using atezolizumab (anti-PD-L1 antibody) and bevacizumab (anti-VEGF antibody) for hepatocellular carcinoma (HCC) was performed between October 2020 and September 2021 at Osaka University Hospital and six related hospitals. Thirty patients who underwent an abdominal MRI examination including chemical-shift imaging (CSI) before starting the combined immunotherapy were retrospectively enrolled in the immunotherapy study. The inclusion criteria were as follows: patients had measurable lesions in the liver, patients did not have marked iron deposition in the liver, and patients had their initial therapeutic response evaluated. The signal intensity of the largest tumor for each patient was acquired by drawing regions of interest (ROI) from both in-phase and opposed-phase images at the same level. The drawing of the ROI was performed by manually drawing the contour of the tumor by using Horos (trademark) software (Nimble Co., LLC, d/b/a Purview). The fat fraction measured by CSI ($FF_{CSI}$) was calculated by the equation (2) given above.

A tumor having a $FF_{CSI}$ of 10% or more was defined as steatotic hepatocellular carcinoma. As a result, seven out of 30 patients were classified as steatotic hepatocellular carcinoma. No significant difference in clinical backgrounds was observed between patients with steatotic hepatocellular carcinoma and nonsteatotic hepatocellular carcinoma. Chemical-shift imaging images of hepatocellular carcinoma having a $FF_{CSI}$ of 35% (FIG. 12A1 and FIG. 12A2), Gd-EOB-DTPA-enhanced MR images (FIG. 12A3 and FIG. 12A4), and a hematoxylin-eosin image of a tumor biopsy specimen (FIG. 12B) are shown.

During atezolizumab plus bevacizumab combined therapy, the patients underwent dynamic contrast-enhanced computed tomography (CT) scans every 6 weeks to evaluate the therapeutic response. The therapeutic response was assessed by using the modified Response Evaluation Criteria in Solid Tumors (mRECIST criteria).

None of the patients with steatotic hepatocellular carcinoma showed disease progression during the 5.4-month observation period. The patients with steatotic hepatocellular carcinoma experienced a significantly longer progression-free survival rate (PFS) than the patients with nonsteatotic hepatocellular carcinoma (FIG. 13A). In FIG. 13A, * represents $p < 0.05$ for nonsteatotic hepatocellular carcinoma vs. steatotic hepatocellular carcinoma. In addition, the patients with steatotic hepatocellular carcinoma had a higher disease control rate (DCR) than the patients with nonsteatotic hepatocellular carcinoma (FIG. 13B). That is, in the patients each having a $FF_{CSI}$ of 10% or more before the start of the atezolizumab plus bevacizumab combined therapy, the DCR {(CR+PR+SD)/(CR+PR+SD+PD)} was 100%, and the treatment was effective for all the patients. Meanwhile, in the patients each having a $FF_{CSI}$ of less than 10% before the start of the combined therapy, the DCR was 56.5%, and almost half of the patients did not achieve sufficient therapeutic efficacy.

It was shown that the patients with steatotic hepatocellular carcinoma had susceptibility to the immune checkpoint inhibitor, and the intratumor fat fraction may become a novel biomarker for predicting the efficacy of immune checkpoint inhibitor therapy for hepatocellular carcinoma.

The inventors of the present invention have revealed that steatotic hepatocellular carcinoma can be identified by chemical-shift imaging. The identification of steatotic hepatocellular carcinoma by chemical-shift imaging is easily clinically applicable when used in combination with gadoxetate sodium-enhanced MRI, which is used to diagnose advanced hepatocellular carcinoma. The patients with steatotic hepatocellular carcinoma, who were identified by the chemical-shift imaging image, experienced a significantly longer progression-free survival rate than the patients with nonsteatotic hepatocellular carcinoma after therapy with an anti-PD-L1 antibody and an anti-VEGF antibody. Those results suggests that intratumor lipid accumulation may be an novel biomarker for predicting the efficacy of immune checkpoint inhibitor therapy for advanced hepatocellular carcinoma.

Industrial Applicability

The efficacy of a drug is predicted in immunotherapy for a patient with liver cancer, and appropriate treatment is provided.

**Claims**

1. A method of testing susceptibility to an immune checkpoint inhibitor, comprising a step of measuring a fat fraction in a liver cancer tissue.

2. The method according to claim 1, wherein the fat fraction is calculated from an image obtained from the liver cancer tissue or a signal for displaying the image obtained from the liver cancer tissue.

3. The method according to claim 2, wherein the image is an MRI image.

4. The method according to claim 3, wherein the MRI image is a chemical-shift imaging image.

5. The method according to any one of claims 1 to 4, further comprising a step of comparing the fat fraction with a reference value, wherein a case in which the fat fraction is equal to or higher than the reference value is determined as having high susceptibility to the immune checkpoint inhibitor or having susceptibility to the immune checkpoint inhibitor.

6. The method according to claim 5, wherein the reference value is a value selected from a range of 5% or more and 15% or less.

7. The method according to claim 6, wherein the reference value for the fat fraction measured by chemical-shift imaging is 10%.

8. The method according to claim 1, wherein the immune checkpoint inhibitor is an anti-PD-L1 antibody or an anti-PD-1 antibody.

9. The method according to claim 1, wherein the liver cancer tissue is a hepatocellular carcinoma tissue.

10. An apparatus for testing susceptibility to an immune checkpoint inhibitor, comprising:

a signal detection unit configured to detect an MR signal of a liver cancer tissue;
a fat fraction calculation unit configured to calculate a fat fraction in the liver cancer tissue from the MR signal having been detected; and
an output unit configured to output a susceptibility level to the immune checkpoint inhibitor based on comparison between the fat fraction having been calculated and a reference value.

11. A program for testing susceptibility to an immune checkpoint inhibitor, the program causing a computer to execute the steps of:

inputting data on an MR signal of a liver cancer tissue;
calculating a fat fraction in the liver cancer tissue from the data on the MR signal having been input; and
outputting a susceptibility level based on comparison between the fat fraction having been calculated and a reference value.

FIG.1

FIG.2

FIG.3

FIG.4

FIG.5

FIG.6

A

☒ Cluster 1
■ Cluster 2
▤ Cluster 3
▨ Cluster 4
▥ Cluster 5
▦ Cluster 6

B

FIG.7

FIG.8

A

PA     −                              +

B

C

FIG.9

A

B

C

FIG.10

FIG.11

FIG.12

A

B

FIG.13

**A**

**B**

| Antitumor effect | non-Steatotic HCC | Steatotic HCC | *P* value |
|---|---|---|---|
| CR/PR/SD/PD | 0/6/7/10 | 0/2/5/0 | |
| DCR (%) | 56.5 | 100 | 0.064 |

## INTERNATIONAL SEARCH REPORT

International application No.

**PCT/JP2023/016673**

| A. | CLASSIFICATION OF SUBJECT MATTER |
|---|---|

*C12Q 1/06*(2006.01)i; *A61B 5/055*(2006.01)i; *C12M 1/34*(2006.01)i; *G01N 33/48*(2006.01)i; *G01N 33/92*(2006.01)i; *G16H 30/00*(2018.01)i

FI: C12Q1/06; C12M1/34 Z; A61B5/055 311; G16H30/00; G01N33/92; G01N33/48 M

According to International Patent Classification (IPC) or to both national classification and IPC

| B. | FIELDS SEARCHED |
|---|---|

Minimum documentation searched (classification system followed by classification symbols)

C12Q1/06; A61B5/055; C12M1/34; G01N33/48; G01N33/92; G16H30/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2021-012102 A (UNIV KYOTO) 04 February 2021 (2021-02-04) claims 1, 4, 5, 9, examples, etc. | 1-11 |
| A | AHMED, M. et al. Association between body mass index, dosing strategy, and efficacy of immune checkpoint inhibitors. Journal for ImmunoTherapy of Cancer. 2021, vol. 9, e002349 (9 pages), Supplemental material, <doi: 10.1136/jitc-2021-002349> abstract, etc. | 1-11 |
| A | CORTELLINI, A. et al. A multicenter study of body mass index in cancer patients treated with anti-PD-1/PD-L1 immune checkpoint inhibitors: when overweight becomes favorable. Journal for ImmunoTherapy of Cancer. 2019, vol. 7, 57 (11 pages), <doi.org/10.1186/s40425-019-0527-y> abstract, etc. | 1-11 |
| A | KICHENADASSE, G. et al. Association Between Body Mass Index and Overall Survival With Immune Checkpoint Inhibitor Therapy for Advanced Non-Small Cell Lung Cancer. JAMA Oncology. 2020, vol. 6, no. 4, pp. 512-518, <doi:10.1001/jamaoncol.2019.5241> abstract, etc. | 1-11 |

✓ Further documents are listed in the continuation of Box C.    ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2023/016673**

## C. DOCUMENTS CONSIDERED TO BE RELEVANT

| Category* | Citation of document, with indication, where appropriate, of the relevant passages | Relevant to claim No. |
|---|---|---|
| A | JHA, R. C. et al. Small Hepatocellular Carcinoma: MRI Findings for Predicting Tumor Growth Rates. Academic Radiology. 2014, vol. 21, pp. 1455-1464, <http://dx.doi.org/10.1016/j.acra.2014.06.011><br>p. 1456, right column, the last paragraph to p. 1457, left column | 1-11 |
| P, X | MURAI, H. et al. Multiomics identifies the link between intratumor steatosis and the exhausted tumor immune microenvironment in hepatocellular carcinoma. Hepatology. 14 May 2022, vol. 77, pp. 77-91, <DOI: 10.1002/hep.32573><br>INTRODUCTION, the last paragraph, fig. 6, etc. | 1-11 |

Form PCT/ISA/210 (second sheet) (January 2015)

INTERNATIONAL SEARCH REPORT
Information on patent family members

International application No.

**PCT/JP2023/016673**

| Patent document cited in search report | | | Publication date (day/month/year) | Patent family member(s) | Publication date (day/month/year) |
|---|---|---|---|---|---|
| JP | 2021-012102 | A | 04 February 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022074111 A


**Non-patent literature cited in the description**

- *CA Cancer J Clin.*, November 2018, vol. 68 (6), 394-424
- *Cancer Res.*, 2013, vol. 190, 21-32
- *Gastroenterology*, July 2019, vol. 157 (1), 54-64
- *Liver Int.*, October 2013, vol. 33 (9), 1420-7
- *World J Hepatol.*, 27 June 2013, vol. 5 (6), 311-322
- *Nat Genet.*, May 2015, vol. 47 (5), 505-511
- *Cancer Res.*, 15 September 2009, vol. 69 (18), 7385-92
- *Cancer Res.*, 15 August 2008, vol. 68 (16), 6779-88
- *Immunotherapy.*, June 2016, vol. 8 (7), 821-37
- *N Engl J Med.*, 14 May 2020, vol. 382 (20), 1894-1905
- *J Hepatol.*, February 2020, vol. 72 (2), 215-229
- *Gastroenterology*, September 2017, vol. 153 (3), 812-826
- *EClinical Medicine*, 16 September 2021, vol. 41, 101134
- *JAMA Oncol.*, 01 August 2019, vol. 5 (8), 1195-1204
- *JAMA Oncol*, 01 August 2019, vol. 5 (8), 1195-1204
- *Magn Reson Med Sci.*, 2011, vol. 10 (1), 41-8
- *Radiographics*, January 2009, vol. 29 (1), 231-60
- *Proc Natl Acad Sci USA.*, 2018, vol. 115, E10417-E10426
- *Gastroenterology.*, June 2010, vol. 138 (7), 2487-98
- *Bioinformatics.*, 15 July 2009, vol. 25 (14), 1754-60
- *Bioinformatics*, 15 August 2009, vol. 25 (16), 2078-9
- *Genome Res.*, March 2012, vol. 22 (3), 568-76
- *Nucleic Acids Res.*, September 2010, vol. 38 (16), e164
- *Wellcome Open Res.*, 25 November 2016, vol. 1, 20
- *Gastroenterology.*, September 2017, vol. 153 (3), 812-826
- *Hepatology*, December 2016, vol. 64 (6), 2038-2046
- *Gastroenterology*, August 2016, vol. 151 (2), 324-337
- *Bio Protoc.*, 05 May 2020, vol. 10 (9), e3613
- *Can J Biochem Physiol.*, August 1959, vol. 37 (8), 911-7
- *Cell.*, 15 June 2017, vol. 169 (7), 1327-1341
- *Clin Cancer Res.*, 01 April 2019, vol. 25 (7), 2021-2023
- *Nat Med.*, October 2010, vol. 16 (10), 1147-51
- *Nat Commun*, 2013, vol. 4, 2612
- *Cancer Cell.*, 09 April 2018, vol. 33 (4), 547-562
- *Immunity.*, 18 September 2014, vol. 41 (3), 427-439
- *Radiographics.*, January 2009, vol. 29 (1), 231-60